(19) Europäisches Patentamt
European Patent Office
Office européen des brevets

(11) Publication number: **0 055 875**
**B1**

(12) **EUROPEAN PATENT SPECIFICATION**

(45) Date of publication of patent specification: **20.08.86**

(21) Application number: **81201276.3**

(22) Date of filing: **19.11.81**

(51) Int. Cl.[4]: **C 07 C 67/38,** C 07 C 51/54, C 07 C 53/122, C 07 C 69/24, C 07 C 69/30, C 07 C 69/34, C 07 C 69/533, B 01 J 31/28

(54) **Process for the carbonylation of olefins.**

(30) Priority: **06.01.81 GB 8100275**

(43) Date of publication of application:
**14.07.82 Bulletin 82/28**

(45) Publication of the grant of the patent:
**20.08.86 Bulletin 86/34**

(84) Designated Contracting States:
**BE DE FR GB IT NL**

(56) References cited:
**GB-A-2 014 136**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Drent, Eit**
**Badhuisweg 3**
**NL-1031 CM Amsterdam (NL)**

(74) Representative: **Aalbers, Onno et al**
**P.O. Box 302**
**NL-2501 CH The Hague (NL)**

**EP 0 055 875 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a process for the carbonylation of olefins.

Many processes have been described in which olefins are carbonylated in the presence of water, alcohols or carboxylic acids to produce carboxylic acids, esters or anhydrides, respectively. For example, US 3168553 discloses that carbonylation can be effected by the use of a complex catalyst comprising cobalt, ruthenium, rhodium or iridium together with a tri-organophosphine. However, in this process, extremely high pressures are required, and complex mixtures of products are formed. For example when using the preferred catalyst, $Co_2(CO)_8$, in the reaction of ethanol with ethylene, the yield of the desired product, ethyl propionate, is always very much less than the yield of other products.

US 3917677 discloses that the use of halogen-free rhodium catalysts in the presence of tri-organophosphines can be used to produce esters by the carbonylation of olefins in the presence of alcohols. Using this system, relatively high yields of ester are formed, but when using alkenes having 3 or more carbon atoms, the relative amounts of terminal and internal esters are similar, with the internal esters usually predominating. The best ratio of terminal:internal ester obtained is 60:40. This specification also provides comparative data which indicate that the replacement of the rhodium catalyst by palladium produces no carbonylation reaction at all.

Attempts to use palladium catalysts, which are rather cheaper than rhodium catalysts, have been made. German Offenlegungsschrift No. 2410246 discloses that if a zero-valent palladium tri-organophosphine complex is used as catalyst in the presence of a large excess of tri-organophosphine, carbonylation reactions will occur. However, relatively high pressures are required, the cost of the excess phosphine is high, and the process is not entirely satisfactory when olefins having internal double bonds are used as feedstock.

US specification 3437676 also describes the use of specific palladium catalysts. The Examples in this US specification show that the proportion of terminal product as opposed to internal product is generally very low, and that very high pressures are required to ensure efficient reaction.

It has now been found that by the selection of very specific catalyst components, many of the disadvantages of the prior art can be overcome.

The invention therefore provides a process for the carbonylation of an olefin with carbon monoxide in the presence of water, an alcohol or a carboxylic acid, characterized by conducting the reaction in the presence of an essentially halide-free palladium catalyst and a tri-organophosphine and in the absence of sodium acetate and an N-heterocyclic amine base, the tri-organophosphine containing at least one aliphatic carbon atom bound to phosphorus and the molar ratio of the organophosphine to palladium being less than 10:1.

For the UK Patent Application 2014136 is disclosed a process for the preparation of esters, in which a conjugated diene is reacted with a hydroxylated co-reactant in the presence of a palladium salt, a tertiary phosphorous compound and a nitrogen-containing heterocyclic amine base. In the absence of such a base the application reveals that hardly any esters are produced. It is thus very surprising that the process according to our invention either in the presence of weak acids or in the absence of weak acids works very well.

A wide range of olefins can be carbonylated using the process according to the invention, for example those having from 2 to 30, especially 2 to 20, carbon atoms. The olefin may be an unsubstituted alkene, or it may contain one or more substituents. Inert substituents which may be present include halogen atoms and cyano, ester, alkoxy and aryl groups. In addition, the olefin may contain one or more substituents which are not inert under the reaction conditions, for example hydroxy or carboxy groups. The fate of such groups will depend on the precise reaction conditions: for example, a carboxy substituted olefin may be carbonylated in the presence of an alcohol, in which case the main products are compounds in which the acid group has been converted into an ester group by reaction with the alcohol. Alternatively, under some reaction conditions, the carboxy group may actually take part in the carbonylation of the olefin function of a second molecule of olefinic acid. One or more double bonds may be present in any position in the olefin carbon chain. Unsubstituted alkenes with one or two double bonds are preferred, and straight-chain alkenes are especially preferred. A mixture of different olefins can of course be used.

Carbonylation reactions using olefins with more than two carbon atoms in the alkene chain can of course lead to a variety of products. For example, the reaction of propene with water could lead to propane-1-carboxylic acid or to propane-2-carboxylic acid. It is an advantage of the present invention that when using an olefin with more than two carbon atoms in the alkene chain, a high proportion of the product usually contains a terminal carbonyl group, regardless of the position of the double bond(s) in the starting material.

A wide range of alcohols and carboxylic acids may be used as reactant in the process of the invention. For example, the compound may be aliphatic, cycloaliphatic or aromatic, and may carry one or more inert substituents, for example those described above for the olefinic reactant. The compound suitably contains up to 20 carbon atoms. One or more hydroxy and/or carboxy groups may be present, in which case different products can be obtained as desired depending upon the molar ratio of reactants used. For example, a trihydric alcohol can be reacted with a small quantity of olefin to produce a mono-ester, or with a large quantity of olefin to produce a tri-ester.

Thus the choice of hydroxylic compound depends solely on the desired product. The use of water produces carboxylic acids as the initial product. The use of alcohols produces esters, and these can of course be poly-esters as described above. Alkanols such as methanol, ethanol, propanol or trimethylolpropane, and alcohols containing ether linkages, for example triethylene glycol, all produce useful products. The use of carboxylic acids produces acid anhydrides. If an alkanoic acid having n+1 carbon atoms is reacted with an alkene having n carbon atoms, a symmetrical anhydride is produced. Otherwise a mixed anhydride is initially produced.

However, depending on the reaction conditions, further reactions may take place. For example, if an olefin is reacted with water and carbon monoxide, the carboxylic acid produced may react with more olefin and carbon monoxide to produce an anhydride. An ester produced may be hydrolyzed if water is present in the reaction mixture, to form an acid and an alcohol, which may in turn react with further olefin. Similarly, an anhydride may be hydrolysed to form an acid or acids. Thus careful control of the process according to the invention means that a wide range of products can be produced as desired.

If it is desired to prepare a particular acid by the process according to the invention, it may for example be convenient to react one mole of said acid with the corresponding olefin having one less carbon atom to produce the symmetric anhydride; to hydrolyse this anhydride to produce two moles of acid; and to recycle one mole of said acid back to the first stage of the process.

When an alcohol is used as the main hydroxylic reactant in the process of the invention, it has, surprisingly, been found that the addition of small quantities of water and/or an acid, especially a carboxylic acid, to the reaction mixture has a strongly promoting effect on the rate of the reaction of the olefin with the alcohol. Suitable carboxylic acids include alkanoic acids having up to 12 carbon atoms. The water and/or acid is suitably added in an amount of from 1 to 5 mole percent based upon the number of moles of olefin. Hydrohalic acids should preferably not be added to the reaction mixture as they are a source of free halogen and have a detrimental effect upon the reaction, as described below. A similar promoting effect is obtained by the addition of water to a system in which an acid is the principle reactant, and by the addition of an acid to a system in which water is the principle reactant.

The palladium catalyst used in the process according to the invention may be homogeneous, heterogeneous, or partially homogeneous and partially heterogeneous, and should be essentially free from halide ions. Suitable homogeneous catalyst components include salts of palladium with mineral acids other than hydrohalic acids, for example nitrates, sulphates or perchlorates, or with organic acids, for example alkanoic acids having up to 12 carbon atoms. Palladium acetate is an especially suitable homogeneous catalyst. Heterogeneous catalysts comprise metallic palladium or palladium salts deposited on an inert carrier material, for example palladium charcoal. Generally the use of a homogeneous catalyst is preferred, since such catalysts tend to be rather more active than heterogeneous catalysts. However, when using as reactant a long-chain olefin, for example one having more than 6 carbon atoms, and having an internal rather than a terminal double bond, the proportion of product having a terminal carbonyl group tends to be greater when using a heterogeneous catalyst. This catalyst may be used alone under such conditions, but is preferably used in admixture with a homogeneous catalyst which tends to increase the rate of the reaction.

The amount of palladium catalyst used is not critical. Generally from about 0.0001 to 10 moles of palladium are used per mole of olefinic double bond to be carbonylated, the preferred range being from 0.01 to 5 moles per mole.

It may be desirable to add a co-catalyst to the system. It has been found that the use of a cobalt-containing catalyst in conjunction with the palladium catalyst is advantageous, particularly for the preparation of terminally functionalised compounds from olefins having an internal double bond.

The tri-organophosphine promoter used in the process according to the invention must have at least one aliphatic carbon atom bonded to phosphorus. Suitable phosphines include those of the general formula

$$PR^1R^2R^3 \qquad (I)$$

in which each of $R^1$ and $R^2$ independently represents an optionally substituted alkyl, cycloalkyl or aryl group, or together represent an optionally substituted alkylene group, and $R^3$ represents an optionally substituted alkyl or cycloalkyl group. Preferably any alkyl group has up to 20 carbon atoms, any cycloalkyl group preferably has from 5 to 7 carbon atoms, and any aryl group preferably has up to 18 carbon atoms; phenyl groups are preferred aryl groups. Preferably an alkylene group has from 4 to 9, especially 6 to 8, carbon atoms, and such a group may form a monocyclic or a bicyclic ring containing the phosphorus atom.

Possible optional substituents include those given above for the olefinic reactant. Preferably however $R^1$, $R^2$ and $R^3$ are hydrocarbon groups. Preferably $R^3$ is an unsubstituted alkyl or cycloalkyl group, an alkyl group substituted by a phenyl group, or an alkyl group substituted by a group $—PR^1R^2$ where $R^1$ and $R^2$ have the above meanings. Typical groups $R^3$ include methyl, ethyl, propyl, isopropyl, butyl, lauryl, cyclopentyl, cyclohexyl, benzyl, 2-phenylethyl and $—CH_2—P(phenyl)_2$.

Especially preferred phosphines are those of formula I in which at least one and preferably both of $R^1$ and $R^2$ are bound to the phosphorus atom by an aliphatic carbon atom. For example, $R^1$ and $R^2$ may have one of the preferred meanings given above for $R^3$. For economic reasons, it may be preferred to use a phosphine in which all of $R^1$, $R^2$ and $R^3$ represent the same group, especially an unsubstituted alkyl group.

Thus typical phosphines include trimethylphosphine, triethylphosphine, tributylphosphine, $(phenyl)_2P—CH_2—P(phenyl)_2$, and cyclic structures such as:

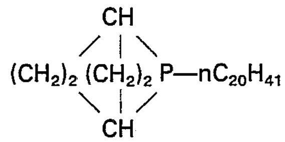

The molar ratio of the tri-organophosphine to palladium (calculated as atomic palladium) must be less than 10:1, and is preferably in the range of from 2:1 to 6:1.

The carbon monoxide required for the reaction may be supplied in substantially pure form, or diluted with any inert gas, for example nitrogen. The presence of more than minor amounts of hydrogen in the gas stream is undesirable since hydrogenation of the olefin then takes place under the reaction conditions. Generally it is preferred that the amount of hydrogen in the gas stream should be less than 5% by volume.

The process according to the invention is carried out using an essentially halide-free palladium catalyst, and preferably the reaction system as a whole is substantially free from halide, and from compounds which generate halide under the reaction conditions. The presence of halide in commercial plants is most undesirable because of the highly corrosive nature of halide. Moreover, the presence of halide ions exerts an inhibiting effect on the process according to the present invention, and is thus not to be recommended.

US patent Specification No. 3917677, which claims the use of rhodium catalysts in the carbonylation of olefins, provides comparative data which show that the carbonylation of ethylene in the presence of methanol, tributylphosphine, sodium acetate (as a buffer) and either $Pd(triphenylphosphine)_3Cl_2$ or palladium acetate, gives no reaction at all. This is attributed to the inactivity of the palladium catalyst. Thus the activity of the specific sodium acetate free palladium catalyst systems used in the process of the present invention is contrary to expectation. The reason for the inhibiting effect of sodium acetate in the prior art process is not understood. However, without being bound by any particular theory, it is believed that the presence of the sodium acetate, a buffer, leads to basic species under the reaction conditions. As stated above, the present process is promoted by the presence of acid. It is therefore believed to be desirable that basic compounds should not be present in the reaction mixture.

The process according to the invention permits the use of very mild reaction conditions. Temperatures in the range of from 100 to 200°C, especially 120 to 150°C, are generally suitable. The pressure may vary over a wide range. Generally, a pressure in the range of from 25 to 100 bar is suitable, with pressures of from 30 to 60 bar being preferred. Higher pressures may of course be used, but are usually economically unattractive.

The molar ratio of the reactants is not critical, and generally the molar ratio of hydroxy groups to olefinic bonds may be in the range of from 0.1:1 to 10:1. When using a mono-olefin and either water, a mono-hydric alcohol or a mono-basic acid, it is usually preferred to use an excess of the hydroxy compound, but as discussed above, if using a poly-hydric alcohol or a poly-basic acid to prepare a poly-ester or a poly-anhydride, it is usually necessary to use an excess of the olefin.

A separate solvent is not essential in the process according to the invention, and often a large excess of one of the reactants, usually the hydroxylic reactant, may form a convenient reaction medium. However, it may in some cases be desirable to use a separate solvent, and any suitable inert medium may be used. A suitable solvent may for example be selected from sulphoxides and sulphones, for example dimethylsulphoxide, diisopropylsulphone or sulpholane, ketones, for example acetone or methyl isobutyl ketone, and ethers, for example diisopropylether. It is often convenient to use as solvent the compound which is the initial reaction product of the carbonylation reaction.

The following Examples illustrate the invention. Unless otherwise specifically stated, all the experiments were carried out in the following manner. A 300 ml magnet-driven autoclave of Hastelloy C (Trade Mark) was charged with 50 ml methanol and the other stated liquid and solid components of the reaction mixture, flushed with carbon monoxide, and then pressurized with further carbon monoxide and any other stated gaseous component. The autoclave was then heated to a temperature of 135°C, and maintained at that temperature for 15 hours. After this time the contents of the autoclave were analysed by gas-liquid chromatography.

Example 1

The autoclave was charged with 0.7 mmol palladium acetate and 4 mmol tri-n-butylphosphine, and pressurized with 20 bar ethene and 20 bar CO. 34% by weight of the resulting reaction mixture was methyl propionate, the only significant by-product being 0.2% by weight of methyl acetate.

Example 2 (Comparison)

Example 1 was repeated exactly except that the tri-n-butylphosphine was replaced with 4 mmol triphenylphosphine. The reaction mixture contained only 5% by weight of methyl propionate.

Examples 3 to 8

The olefin used was propene at a pressure of 8 bar, and CO was added at a pressure of 30 bar. The results obtained are tabulated below. The components present in the system are given in units of mmol unless otherwise stated, and the ester production rate is in units of ester produced per gram palladium (calculated as atomic palladium) per hour.

| Example No. | Components present (mmol) | Ester production rate (g/gPd/h) | % Terminal ester |
|---|---|---|---|
| 3 | 0.7 $Pd(CH_3CO_2)_2$<br>4 $P(nBu)_3$ | 3 | 73 |
| 4 | 1.4 $Pd(CH_3CO_2)_2$<br>4 $[(phenyl)_2P]_2CH_2$ | 5 | 80 |
| 5 | 0.7 $Pd(CH_3CO_2)_2$<br>4 eicosyl phosphabicyclononane | 4 | 80 |
| 6 | 0.7 $Pd(CH_3CO_2)_2$<br>4 $P(nBu)_3$<br>40 $CH_3CO_2H$ | 11 | 75 |
| 7 | 0.7 $Pd(CH_3CO_2)_2$<br>4 $P(nBu)_3$<br>150 $H_2O$ | 12 | 75 |
| 8 | 0.7 $Pd(CH_3CO_2)_2$<br>4 $P(nBu)_3$<br>10 g dimethylsulphone | 5 | 80 |

In all these Examples, the products were exclusively esters of $C_4$ acids, with no detectable side-products being obtained.

The results of Examples 6 and 7 show that the addition of water or acetic acid to the reaction mixture increases the rate of production of ester considerably without having any marked detrimental effect on selectivity.

Example 9 (Comparison)

The procedure of Example 3 was repeated exactly except that an amount of 880 mg sodium acetate was added to the reaction mixture. No reaction at all took place, as expected from the result of Example 5 of US Patent 3917677.

Examples 10 to 12

10 ml of $C_5$ olefin were used as reactant, with a CO pressure of 30 bar. The results are tabulated below. The only products obtained were esters.

| Example No. | Components present, mmol | Olefin | Ester production rate g/g Pd/h | Terminal ester, % |
|---|---|---|---|---|
| 10 | 0.7 $Pd(CH_3CO_2)_2$<br>4 $P(nBu)_3$<br>150 $H_2O$ | pentene-1 | 10 | 79 |
| 11 | 0.7 $Pd(CH_3CO_2)_2$<br>4 $P(nBu)_3$<br>150 $H_2O$ | pentene-2 | 2.5 | 70 |
| 12 | 0.7 $Pd(CH_3CO_2)_2$<br>4 $P(nBu)_3$<br>150 $H_2O$<br>0.35 $Co_2(CO)_8$ | pentene-2 | 7 | 72 |

Examples 13 and 14

The olefin reactant was 10 ml *cis* hexene-3, and CO pressure was 30 bar. The results are tabulated below.

| Example No. | Components present, mmol | Ester production rate, g/g Pd/h | Terminal ester, % |
|---|---|---|---|
| 13 | 0.7 $Pd(CH_3CO_2)_2$<br>4 $P(nBu)_3$<br>150 water | 2 | 72 |
| 14 | 0.7 $Pd(CH_3CO_2)_2$<br>4 $P(nBu)_3$<br>40 $CH_3CO_2H$ | 5 | 75 |

Example 15 (Comparison)

Example 13 was repeated except that the water was replaced by 10% HCl solution. Only 43% of the product obtained was terminal ester, illustrating that the presence of halide has a marked effect upon the reaction.

Example 16

The catalyst used was 0.7 mmol palladium acetate and 4 mmol tri-n-butylphosphine, the olefin used was 10 ml octene-1, and a CO pressure of 20 bar was used. The selectivity to methyl esters of $C_9$ acids was 100%, the ester production rate was 3 g/gPd/h, and the ester product contained 70% of terminal ester.

Example 17 to 22

A series of experiments were carried out using $C_{12}$ mono-olefins with either a terminal or an internal double bond. 10 ml olefin were used in each case, the CO pressure being 30 bar. Example 17 was carried out using only 25 ml methanol instead of 50 ml. The results are tabulated below.

| Example No. | Components present, mmol | Olefin double bond position | Conversion of olefin to ester, % | Terminal ester, % |
|---|---|---|---|---|
| 17 | 0.7 $Pd(CH_3CO_2)_2$<br>4 $P(nBu)_3$<br>150 $H_2O$<br>25 ml sulpholane | terminal | 31 | 71 |
| 18 | 0.7 $Pd(CH_3CO_2)_2$<br>4 $P(nBu)_3$<br>50 $CH_3CO_2H$ | terminal | 54 | 69 |
| 19 | 0.7 $Pd(CH_3CO_2)_2$<br>4 $P(nBu)_3$<br>50 octanoic acid<br>0.5 g of 3% w palladium on charcoal | terminal | 70 | 74 |
| 20 | 4 $P(nBu)_3$<br>50 $CH_3CO_2H$<br>0.2 g of 10% w palladium on charcoal | terminal | 46 | 75 |
| 21 | 0.7 $Pd(CH_3CO_2)_2$<br>4 $P(nBu)_3$<br>50 $CH_3CO_2H$<br>0.5 g of 3% w palladium on charcoal | terminal | 64 | 73 |
| 22 | 0.7 $Pd(CH_3CO_2)_2$<br>4 $P(nBu)_3$<br>50 $CH_3CO_2H$<br>0.5 g of 3% w palladium on charcoal | internal | 26 | 68 |

Example 23

Example 18 was repeated using a higher CO pressure of 50 bar. The conversion increased from 54 to 65% and the percentage of terminal ester in the product increased from 69 to 74%.

Example 24 (Comparison)

Example 21 was repeated exactly except that the 4 mmol tri-n-butylphosphine were replaced by 4 mmol triphenylphosphine. The conversion of olefin dropped from 64 to 29% and the percentage of terminal ester dropped from 73 to 56%.

Examples 25 to 27 (Comparison)

Experiments were carried out to investigate the effects of using large quantities of triphenylphosphine. 10 ml of $C_{12}$ mono-olefin were used in each case. The CO pressure was 30 bar.

| Example No. | Components present (mmol) | Olefin double bond position | Conversion of olefin (%) | % of terminal ester |
|---|---|---|---|---|
| 25 | 0.7 $Pd[P(phenyl)_3]_4$<br>9 $P(phenyl)_3$ | internal | 6 | 48 |
| 26 | 0.7 $Pd[P(phenyl)_3]_4$<br>9 $P(phenyl)_3$<br>0.5 g of 3% w palladium in charcoal | internal | 18 | 42 |
| 27 | 0.7 $Pd(CH_3CO_2)_2$<br>9 $P(phenyl)_3$<br>150 $H_2O$ | terminal | 4 | 45 |

Example 28 (Comparison)

An experiment was carried out using a cobalt catalyst with 10 ml of a $C_{12}$ olefin with a terminal double bond and a CO pressure of 30 bar. The catalyst consisted of 0.7 mmol $Co(CH_3CO_2)_2$ and 3 mmol alpha-picoline. The conversion of olefin was high, 59%, but the percentage of terminal ester was very low, only 37%. Substantial quantities of alkane and aldehyde were formed as by-products.

Example 29 (Comparison)

An experiment was carried out using a rhodium catalyst, with propene at 10 bar pressure as the olefin, and a CO pressure of 30 bar. The catalyst consisted of 0.7 mmol of $Rh.(CH_3.CO.CH.CO.CH_3).(CO)_2$ and 4 mmol tri-n-butylphosphine. The proportion of methyl butyrate amongst the products formed was only 45% molar, the remainder being largely ketones and methyl formate.

Example 30 (Comparison)

0.7 g of 10% w palladium charcoal catalyst was used, along with 50 mmol acetic acid, 10 ml of internal $C_{12}$ mono-olefin, and a CO pressure of 30 bar. However, the $P(nBu)_3$ concentration was increased to 14 mmol. A high proportion of terminal ester, 65%, was produced, but the conversion of olefin dropped to only 10%.

Example 31

An experiment was carried out in which the 50 ml methanol was entirely replaced by 50 ml acetic acid. The catalyst used was 0.7 mmol palladium acetate, 4 mmol tri-n-butylphosphine, and 0.5 g of 3% w palladium on charcoal. The olefin used was propene at a pressure of 8 bar, and the CO pressure was 30 bar. A mixture of anhydrides containing n-butyryl, i-butyryl and acetyl moieties was produced at a rate of 10g/gPd/h. The ratio of n-butyryl moieties to i-butyryl moieties was 1.2:1.

Example 32

Example 31 was repeated except that the 50 ml acetic acid was replaced by 20 ml acetic acid and 25 ml sulpholane, and that 6.5 mmol tri-n-butylphosphine were used. Conversion of acetic acid to products was 35%, and the molar ratio of n-butyric acid residues to i-butyric acid residues in the product was 2:1.

Example 33

An experiment was carried out in which the 50 ml methanol was entirely replaced by a solution of 2.4 g trimethylol propane in 25 ml sulpholane as solvent. The catalyst was 0.7 mmol palladium acetate, 6.5 mmol tri-n-butylphosphine and 7 mmol propionic acid. Ethene at a pressure of 15 bar and CO at a pressure of 30 bar were introduced. After the 15 hours reaction time the reaction mixture was analysed. 86% of the alcohol had been converted to tri-ester. No mono- or di-ester was observed.

Example 34

The olefin used in this experiment was 5 ml 4-carboxybut-1-ene, the catalyst was 0.7 mmol palladium acetate and 5 mmol tri-n-butylphosphine, and the CO pressure was 30 bar. After the 15 hours reaction time, none of the olefinic acid remained in the reaction mixture. 50% molar of the product consisted of 4-methoxycarbonylbutenes. The remainder of the product was 1,4-dimethoxycarbonylbutane (70% m), 1,3-dimethoxycarbonylbutane (22% m) and 1,2-dimethoxycarbonylbutane (8% m).

Example 35

The olefin used in this experiment was 10 mls butadiene, the catalyst was 0.7 mmol palladium acetate, 4 mmol tri-n-butylphosphine, and 150 mmol water, and the CO pressure was 30 bar. Analysis of the reaction mixture showed the presence of 30% by weight of methyl 3,8-nonadienoate, plus a substantial amount of butadiene dimer.

**Claims**

1. A process for the carbonylation of an olefin with carbon monoxide in the presence of water, an alcohol, or a carboxylic acid, characterized by conducting the reaction in the presence of an essentially halide-free palladium catalyst and a tri-organophosphine and in the absence of sodium acetate and a N-heterocyclic amine base, the tri-organophosphine containing at least one aliphatic carbon atom bound to phosphorus and the molar ratio of the organophosphine to palladium being less than 10:1.

2. A process as claimed in claim 1, characterized in that the olefin has from 2 to 30 carbon atoms and is unsubstituted or substituted by one or more of the same or different substituents selected from halogen atoms and cyano, ester, alkoxy, aryl, hydroxy and carboxy groups.

3. A process as claimed in claim 2, characterized in that the olefin is an unsubstituted alkene having one or two double bonds.

4. A process as claimed in any one of claims 1 to 3, characterized in that the olefin is carbonylated in the presence of an alcohol or a carboxylic acid having up to 20 carbon atoms.

5. A process as claimed in claim 4, characterized in that the olefin is carbonylated in the presence of a mono- or poly-alkanol, an alcohol containing ether linkages, or a mono- or poly-basic alkanoic acid.

6. A process as claimed in any one of claims 1 to 5, characterized in that the olefin is carbonylated in the presence of water or an alcohol, and there is added to the reaction mixture a carboxylic acid in an amount of from 1 to 5 mole percent based on the number of moles of olefin.

7. A process as claimed in any one of claims 1 to 5, characterized in that the olefin is carbonylated in the presence of an alcohol or a carboxylic acid, and there is added to the reaction mixture, water in an amount of from 1 to 5 mole percent based on the number of moles of olefin.

8. A process as claimed in any one of claims 1 to 7, characterized in that the tri-organophosphine has the general formula

$$PR^1R^2R^3 \qquad (I)$$

in which each of $R^1$ and $R^2$ independently represents an alkyl, cycloalkyl or aryl group, or together represent an alkylene group, and $R^3$ represents an alkyl or cycloalkyl group.

9. A process as claimed in claim 8, characterized in that $R^3$ represents an unsubstituted alkyl or cycloalkyl group, an alkyl group substituted by a phenyl group, or an alkyl group substituted by a group $—PR^1R^2$, where $R^1$ and $R^2$ have the meanings given in claim 8.

10. A process as claimed in either claim 8 or claim 9, characterized in that both of $R^1$ and $R^2$ in the formula I are bound to the phosphorus atom by aliphatic carbon atoms.

11. A process as claimed in any one of claims 1 to 10, characterized in that the reaction is carried out at a temperature in the range of from 100 to 200°C.

**Patentansprüche**

1. Verfahren zur Carbonylierung eines Olefins mit Kohlenmonoxid in Gegenwart von Wasser, einem Alkohol oder einer Carbonsäure, dadurch gekennzeichnet, daß man die Reaktion in Gegenwart eines im wesentlichen halogenfreien Palladiumkatalysators und eines Triorganophosphins und in Abwesenheit von Natriumacetat und einer N-heterocyclischen Aminbase durchführt, wobei das Triorganophosphin mindestens ein aliphatisches Kohlenstoffatom, gebunden an das Phosphoratom enthält und das Molverhältnis von Organophosphin zu Palladium weniger als 10:1 ist.

2. Verfahren nach Anspruch 1, dadurch gekennzeichnet, daß das Olefin 2 bis 30 Kohlenstoffatome besitzt und nicht substituiert oder durch eine oder mehrere gleiche oder unterschiedliche Substituenten, ausgewählt aus Halogenatomen und Cyano-, Ester-, Alkoxy-, Aryl-, Hydroxy- und Carboxygruppen substituiert ist.

3. Verfahren nach Anspruch 2, dadurch gekennzeichnet, daß das Olefin ein unsubstituiertes Alken mit ein oder zwei Doppelbindungen ist.

4. Verfahren nach einem der Ansprüche 1 bis 3, dadurch gekennzeichnet, daß das Olefin in Gegenwart eines Alkohols oder einer Carbonsäure mit bis zu zu 20 Kohlenstoffatomen carbonyliert wird.

5. Verfahren nach Anspruch 4, dadurch gekennzeichnet, daß das Olefin in Gegenwart eines Mono- oder Polyalkanols, eines Alkohols, der Etherbindungen enthält, oder einer ein- oder mehrbasischen Alkansäure carbonyliert wird.

6. Verfahren nach einem der Ansprüche 1 bis 5, dadurch gekennzeichnet, daß das Olefin in Gegenwart von Wasser oder einem Alkohol carbonyliert wird und zu dem Reaktionsgemisch eine Carbonsäure in einer Menge von 1 bis 5 Mol-%, bezogen auf die Anzahl der Mole Olefin, zugegeben wird.

7. Verfahren nach einem der Ansprüche 1 bis 5, dadurch gekennzeichnet, daß das Olefin in Gegenwart eines Alkohols oder einer Carboxylgruppe carbonyliert wird und zu dem Reaktionsgemisch Wasser in einer Menge von 1 bis 5 Mol-%, bezogen auf die Anzahl Mole Olefin, zugesetzt wird.

8. Verfahren nach einem der Ansprüche 1 bis 7, dadurch gekennzeichnet, daß das Triorganophosphin die Formel

$$PR^1R^2R^3 \qquad (I)$$

besitzt, in der $R^1$ und $R^2$ jeweils unabhängig voneinander eine Alkyl-, Cycloalkyl- oder Arylgruppe oder zusammen eine Alkylengruppe bedeuten und $R^3$ eine Alkyl- oder Cycloalkylgruppe ist.

9. Verfahren nach Anspruch 8, dadurch gekennzeichnet, daß $R^3$ eine unsubstituierte Alkyl- oder Cycloalkylgruppe, eine durch eine Phenylgruppe substituierte Alkylgruppe oder eine durch eine Gruppe $—PR^1R^2$ substituierte Alkylgruppe bedeutet, wobei $R^1$ und $R^2$ die in Anspruch 8 angegebene Bedeutung haben.

10. Verfahren nach Anspruch 8 oder Anspruch 9, dadurch gekennzeichnet, daß $R^1$ und $R^2$ in der Formel I beide über aliphatische Kohlenstoffatome an das Phosphoratom gebunden sind.

11. Verfahren nach einem der Ansprüche 1 bis 10, dadurch gekennzeichnet, daß die Reaktion bei einer Temperatur im Bereich von 100 bis 200°C durchgeführt wird.

**Revendications**

1. Procédé de carbonylation d'une oléfine avec du monoxyde de carbone en présence d'eau, d'un alcool, ou d'un acide carboxylique, caractérisé en ce que l'on mène la réaction en présence d'un catalyseur au palladium essentiellement exempt d'halogénure et d'une tri-organophosphine et en l'absence d'acétate de sodium et d'une base amine N-hétérocyclique, la tri-organophosphine contenant au moins un atome de carbone aliphatique lié au phosphore et la proportion molaire de l'organophosphine au palladium étant inférieure à 10:1.

2. Procédé selon la revendication 1, caractérisé en ce que l'oléfine a de 2 à 30 atomes de carbone et est non substituée ou substituée par un ou plusieurs des substituants identiques ou différents choisis parmi les atomes d'halogène et les groupes cyano, ester, alcoxy, aryle, hydroxy et carboxy.

3. Procédé selon la revendication 2, caractérisé en ce que l'oléfine est un alcène non substitué comportant une ou deux doubles liaisons.

4. Procédé selon l'une quelconque des revendications 1 à 3, caractérisé en ce que l'oléfine est carbonylée en présence d'un alcool ou d'un acide carboxylique ayant jusqu'à 20 atomes de carbone.

5. Procédé selon la revendication 4, caractérisé en ce que l'oléfine est carbonylée en présence d'un mono- ou d'un poly-alcanol, d'un alcool contenant des liaisons éther ou d'un mono- ou poly-acide alcanoïque.

6. Procédé selon l'une quelconque des revendications 1 à 5, caractérisé en ce que l'oléfine est carbonylée en présence d'eau ou d'un alcool et qu'on ajoute au mélange réactionnel un acide carboxylique en quantité de 1 à 5 pour cent en moles par rapport au nombre de moles d'oléfine.

7. Procédé selon l'une quelconque des revendications 1 à 5, caractérisé en ce que l'oléfine est carbonylée en présence d'un alcool ou d'un acide carboxylique et qu'on ajoute au mélange réactionnel de l'eau en quantité de 1 à 5 pour cent en moles par rapport au nombre de moles d'oléfine.

8. Procédé selon l'une quelconque des revendications 1 à 7, caractérisé en ce que la tri-organophosphine a pour formule générale

$$PR^1R^2R^3 \qquad (I)$$

dans laquelle chaque $R^1$ et $R^2$ représente indépendamment un groupe alkyle, cycloalkyle ou aryle ou représentent ensemble un groupe alkylène, et $R^3$ représente un groupe alkyle ou cycloalkyle.

9. Procédé selon la revendication 8, caractérisé en ce que $R^3$ représente un groupe alkyle ou cycloalkyle non substitué, un groupe alkyle substitué par un groupe phényle ou un groupe alkyle substitué par un groupe $—PR^1R^2$, dans lequel $R^1$ et $R^2$ ont les significations indiquées dans la revendication 8.

10. Procédé selon l'une quelconque des revendications 8 ou 9, caractérisé en ce que $R^1$ et $R^2$ dans la formule I sont tous deux liés à l'atome de phosphore par des atomes de carbone aliphatiques.

11. Procédé selon l'une quelconque des revendications 1 à 10, caractérisé en ce que la réaction est effectuée à une température dans l'intervalle de 100 à 200°C.